(19) Europäisches Patentamt
European Patent Office
Office européen des brevets

(11) Publication number: **0 320 981**
**A1**

(12) # EUROPEAN PATENT APPLICATION

(21) Application number: **88121136.1**

(51) Int. Cl.⁴: **C07C 43/12 , C07C 41/00**

(22) Date of filing: **16.12.88**

(30) Priority: **18.12.87 JP 322223/87**
**24.12.87 JP 333577/87**

(43) Date of publication of application:
**21.06.89 Bulletin 89/25**

(84) Designated Contracting States:
**DE FR GB IT**

(71) Applicant: **DAIKIN INDUSTRIES LIMITED**
**Umeda Center Building 4-12, Nakazaki-nishi**
**2-chome Kita-ku**
**Osaka-shi Osaka-fu(JP)**

(72) Inventor: **Tomihashi, Nobuyuki**
**20-3, Sakuragaoka Kita-machi**
**Takatsuki-shi Osaka-fu(JP)**
Inventor: **Yamana, Masayuki**
**4-12-17, Shinmori**
**Asahi-ku Osaka-shi Osaka-fu(JP)**
Inventor: **Araki, Takayuki**
**1-17, Tsukide-cho**
**Kadoma-shi Osaka-fu(JP)**

(74) Representative: **Hansen, Bernd, Dr.rer.nat. et al**
**Hoffmann, Eitle & Partner Patentanwälte**
**Arabellastrasse 4 Postfach 81 04 20**
**D-8000 München 81(DE)**

(54) **Fluorine-containing ether and its preparation.**

(57) A fluorine-containing ether of the formula:

$$R_{f1}-\underset{\displaystyle R_{f2}}{\underset{|}{C}H}-(CH_2)_m-O\underset{\displaystyle X}{\underset{|}{C}H}CH_3 \qquad (I)$$

wherein X is a chlorine atom or a bromine atom, and $R_{f1}$ and $R_{f2}$ are the same or different and a hydrogen atom, a fluorine atom or a fluorine-containing $C_1$-$C_{20}$ aliphatic or ether group, m is an integer of 0 to 9 provided that at least one of $R_{f1}$ and $R_{f2}$ is not a hydrogen atom, and when m is zero (0), neither of $R_{f1}$ and $R_{f2}$ is a fluorine atom is prepared by reacting at least one compound selected from the group consisting of a fluorine-containing alcohol of the formula:

$$R_{f1}-\underset{\displaystyle R_{f2}}{\underset{|}{C}H}-(CH_2)_m-OH \qquad (II)$$

EP 0 320 981 A1

or a fluorine-containing acetal of the formula:

$$[R_{f1}-\underset{\displaystyle R_{f2}}{\underset{|}{C}H}-(CH_2)_m-O]_2-CHCH_3 \qquad (III)$$

wherein $R_{f1}$, $R_{f2}$ and m are the same as defined above with acetaldehyde and at least one hydrogen halide selected from the group consisting of hydrogen chloride and hydrogen bromide.

# FLUORINE-CONTAINING ETHER AND ITS PREPARATION

## BACKGROUND OF THE INVENTION

### Field of the Invention

The present invention relates to a process for preparing a fluorine-containing ether and a novel ether. More particularly, the present invention relates to a process for preparing 1-halogenoethyl fluoroalkyl ether and some novel 1-halogenoethyl fluoroalkyl ethers.

### Description of the Related Art

Several processes for preparing fluorine-containing ethers have been proposed. For example, U.S. Patent Nc. 2,830,007 and Zh. Org. Khim., 18, 744-9 (1982) disclose a process for preparing a fluorine-containing ether comprising addition reacting a fluorine-containing alcohol with acetylene. However, this addition reaction should be effected at high temperature under high pressure and produces the product in a low yield.

U.S. Patent No. 2,870,218 discloses a process for preparing a fluorine-containing vinyl ether comprising producing a fluorine-containing acetal and then pyrolyzing the acetal. In this process, since the acetal is pyrolyzed, the process requires high temperature and a life of a catalyst (e.g. alumina and silica) to be used should be shortened.

GB Patent No. 739,731 discloses a process for synthesizing a fluorine-containing vinyl ether comprising ester/ether exchange between vinyl acetate and a fluorine-containing alcohol in the presence of a mercury base catalyst (e.g. $Hg(OAc)_2$). However, the use of $Hg^{2+}$ in the catalyst would induces a pollution problem.

Further, the above described conventional processes are not commercially advantageous processes for preparing the fluorine-containing vinyl ether in high yields. Then, a process for preparing a fluorine-containing vinyl ether from 1-chloroethyl fluoroalkyl ether which is a kind of the fluorine-containing ether is proposed.

U.S. Patent No. 3,658,918 discloses a process for synthesizing 1-chloroethyl fluoroalkyl ether comprising chlorination of fluoroalkyl ethyl ether. However, this process is not suitable for commercial scale production, since synthesis of the fluoroalkyl ethyl ether to be used as the starting material is necessary and in said synthesis, a fluorine-containing carboxylic acid and $LiAlH_4$ should be used. 1,1-Dichloroethyl fluoroalkyl ether or fluorine-containing acetals are by-produced so that the yield of the desired 1-chloroethyl fluoroalkyl ether is decreased. This U.S. Patent also discloses a fluorine-containing vinyl ether having four or more carbon atoms in the fluoroalkyl group which can be prepared by dehydrogen chlorination of the 1-chloroethyl fluoroalkyl ether. However, the prepared fluorine-containing ether having the long fluoroalkyl chain in the molecule provides a polymer which has poor solubility in a solvent such as acetate type solvents and aromatic solvents or less compatibility with hydrocarbon base polymers (e.g. methyl methacrylate polymers) which are usually used as a solvent type coating polymer. Therefore, the fluorine-containing vinyl ether disclosed in said U.S. Patent has limited application fields and is not suitable as a monomer of a solvent type cold setting polymer (see Table below).

## SUMMARY OF THE INVENTION

One object of the present invention is to provide a process for preparing a fluorine-containing ether, which can be an intermediate in the preparation of a fluorine-containing vinyl ether, from an easily available starting material in a high yield under mild conditions.

Another object of the present invention is to provide a novel fluorine-containing ether.

According to the first aspect of the present invention, there is provided a process for preparing a fluorine-containing ether of the formula:

$$R_{f1}-\underset{\displaystyle R_{f2}}{\underset{|}{CH}}-(CH_2)_m-O\underset{\displaystyle X}{\underset{|}{C}}HCH_3 \qquad (I)$$

wherein X is a chlorine atom or a bromine atom, and $R_{f1}$ and $R_{f2}$ are the same or different and a hydrogen atom, a fluorine atom or a fluorine-containing $C_1$-$C_{20}$ aliphatic or ether group, m is an integer of 0 to 9 provided that at least one of $R_{f1}$ and $R_{f2}$ is not a hydrogen atom, and when m is zero (0), neither of $R_{f1}$ and $R_{f2}$ is a fluorine atom, which process comprising reacting at least one compound selected from the group consisting of a fluorine-containing alcohol of the formula:

$$R_{f1}-\underset{\displaystyle R_{f2}}{\underset{|}{CH}}-(CH_2)_m-OH \qquad (II)$$

or a fluorine-containing acetal of the formula:

$$[R_{f1}-\underset{\displaystyle R_{f2}}{\underset{|}{CH}}-(CH_2)_m-O]_2-CHCH_3 \qquad (III)$$

wherein $R_{f1}$, $R_{f2}$ and m are the same as defined above with acetaldehyde and at least one hydrogen halide selected from the group consisting of hydrogen chloride and hydrogen bromide.

According to the second aspect of the present invention, there is provided a novel fluorine-containing ether of the formula:

$$R_{f3}-\underset{\displaystyle R_{f4}}{\underset{|}{CH}}-(CH_2)_n-O\underset{\displaystyle X}{\underset{|}{C}}HCH_3 \qquad (I')$$

wherein X is a chlorine atom or a bromine atom, and $R_{f3}$ and $R_{f4}$ are the same or different and a hydrogen atom, a fluorine atom or a fluorine-containing $C_1$-$C_3$ aliphatic group, n is an integer of 0 to 3 provided that at least one of $R_{f3}$ and $R_{f4}$ is not a hydrogen atom and the total number of the carbon atoms in $R_{f3}$ and $R_{f4}$ is not larger than 3.

## DETAILED DESCRIPTION OF THE INVENTION

In the formula (I), $R_{f1}$ or $R_{f2}$ is a hydrogen atom, a fluorine atom or a fluorine-containing $C_1$-$C_{20}$ aliphatic group, but at least one of $R_{f1}$ and $R_{f2}$ is not a hydrogen atom. Specific examples of the fluorine-containing aliphatic group are straight or branched perfluoroalkyl groups such as $CF_3(CF_2)_p$- or $(CF_3)_2CF(CF_2)_p$- (in which p is zero or a positive integer) and fluoroalkyl groups having at least one hydrogen atom on the carbon atom(s) such as $HCF_2(CF_2)_p$-(in which p is the same as defined above) or $CF_3CFHCF_2$-. Specific examples of the fluorine-containing ether group are $F[CF(CF_3)CF_2O]_q$-$CF_2CF_2$-, $F(CF_2CF_2CF_2O)_q$- (in which q is a positive integer) and the like.

Examples of the fluorine-containing alcohol (II) are $CH_2FCH_2OH$, $CHF_2CH_2OH$, $CF_3CH_2OH$, $CF_3CH_2CH_2OH$, $C_2F_5CH_2OH$, $C_3F_7CH_2OH$, $C_2F_5CH_2CH_2OH$, $C_3F_7CH_2CH_2OH$, $C_8F_{17}CH_2CH_2OH$, $CHF_2CF_2CH_2OH$, $H(CF_2CF_2)_2CH_2OH$, $H(CF_2CF_2)_3CH_2OH$, $H(CF_2CF_2)_4CH_2OH$, $H(CF_2CF_2)_5CH_2OH$, $CF_3CHFCF_2CH_2OH$, $(CF_3)_2CHOH$, $(CF_3)_2CHCH_2OH$, $(CF_3)_2CFCF_2CF_2CH_2CH_2OH$, $(CF_3)_2CFCH_2OH$, $CF_3CF_2CF_2OCF_2CF_2CH_2OH$, $F[CF(CF_3)CF_2O]_2CF_2CF_2CH_2OH$, $F[CF(CF_3)CF_2O]_3CF_2CF_2CH_2OH$, $C_3F_7OCF_2CF_2CF_2OCF_2CF_2CH_2OH$, $F(CF_2CF_2CF_2O)_3CF_2CF_2CH_2OH$ and the like.

The fluorine-containing acetal (III) may be prepared by reacting the fluorine-containing alcohol (II) with acetaldehyde including paraldehyde in the presence of an acidic catalyst such as p-toluenesulfonic acid.

Examples of the fluorine-containing acetal (III) are $CH_3CH\text{-}(OCH_2CH_2F)_2$, $CH_3CH\text{-}(OCH_2CF_3)_2$, $CH_3CH\text{-}(OCH_2C_2F_5)_2$, $CH_3CH\text{-}(OCH_2CF_2CHF_2)_2$, $CH_3CH\text{-}(OCH_2CF_2CF_2CF_2CF_2H)_2$, $CH_3CH\ [OCH_2(CF_2CF_2)_3H]_2$, $CH_3CH\text{-}[OCH_2(CF_2CF_2)_5H]_2$, $CH_3CH\text{-}(OCH_2CF_2\ CHFCF_3)_2$, $CH_3CH\text{-}[OCH(CF_3)_2]_2$, $CH_3CH\text{-}(OCH_2C_3F_7)_2$, $CH_3CH(OCH_2CH_2C_2F_5)_2$, $CH_3CH\text{-}(OCH_2CH_2C_8F_{17})_2$, $CH_3CH\text{-}[OCH_2CH_2CF_2CF_2CF(CF_3)_2]_2$, $CH_3CH\text{-}[OCH\text{-}(CF_3)_2]_2$, $CH_3CH\text{-}(OCH_2CF_2CF_2OC_3F_7)_2$, $CH_3CH\text{-}[OCH_2CF_2CF_2OCF_2CF(CF_3)OCF_2CF_2(CF_3)]_2$, $CH_3CH\text{-}[OCH_2CF_2CF_2OCF_2CF(CF_3)OCF_2CF(CF_3)OCF_2CF_2(CF_3)]_2$, $CH_3CH\text{-}(OCH_2CF_2CF_2OCF_2CF_2CF_2OC_3F_7)_2$, $CH_3CH\text{-}(OCH_2CF_2CF_2OCF_2CF_2CF_2OCF_2CF_2CF_2OC_3F_7)_2$ and the like.

As the aldehyde material, not only monomeric aldehyde but also dimeric or trimeric aldehyde (paraldehyde) and a polymer of aldehyde (metaldehyde) can be used. The amount of aldehyde is at least equivalent, preferably 1 to 1.5 times equivalent or in some cases 1.5 to 5 times equivalent based on the total amount of the fluorine-containing alcohol and/or the fluorine-containing acetal.

Hydrogen chloride or hydrogen bromide to be used in the reaction of the present invention contains impurities such as water, chlorine gas and bromine gas as little as possible since they have adverse affects on the reaction. Preferably, gaseous hydrogen chloride or hydrogen bromide with a purity of not lower than 95 % is used, although hydrogen chloride or hydrogen bromide with a purity of not lower than 90 % may be used. When 35 % hydrochloric acid or 47 % hydrobromic acid is used, the desired fluorine-containing ether is produced in a very low yield or no desired ether is produced. The amount of hydrogen chloride or hydrogen bromide is at least equivalent, preferably 1 to 1.5 times equivalent based on the total amount of the fluorine-containing alcohol and/or the fluorine-containing acetal, although 1.5 to 5 times equivalent of hydrogen chloride or hydrogen bromide may be used.

The reaction proceeds in the absence of a solvent, although it can be carried out in a solvent. Specific examples of the solvent are hydrocarbon solvents other than aromatic hydrocarbon solvent (e.g. pentane, hexane, cyclohexane, etc.), chlorine-containing solvents (e.g. dichloromethane, chloroform, carbon tetrachloride, 1,2-dichloroethane, etc.) and flon type solvents (e.g. flon-12, flon-113, flon-114, flon-C318, etc.).

The reaction temperature is usually from -50 to +150 °C, preferably from -20 to +50 °C. The reaction pressure is usually from 0 to 30 $kg/cm^2G$, preferably from atmospheric pressure to 10 $kg./cm^2G$. The reaction time varies with the kinds of the reactants and other reaction conditions such as the reaction temperature and is usually from 0.1 to 20 hours.

The reaction according to the present invention may be carried out by bubbling gaseous hydrogen chloride and/or hydrogen bromide through a mixture of acetaldehyde with the fluorine-containing alcohol (II) and/or the fluorine-containing acetal (III).

The fluorine-containing ether (I) can be easily dehydrogen halogenide to form a fluorine-containing vinyl ether of the formula:

$$CH_2=CHO(CH_2)_m\underset{\displaystyle R_{f2}}{\underset{|}{C}}HR_{f1} \qquad (V)$$

wherein $R_{f1}$ and $R_{f2}$ are the same as defined above. The dehydrogen halogenation can be carried out, for example, by reacting the fluorine-containing ether (I) with a base, preferably a tertiary amine (e.g. trimethylamine, triethylamine, tributylamine, pyridine, N,N-dimethylbenzylamine, N,N-diethylbenzylamine, N,N-dimethylaniline, N,N-dimethylcyclohexylamine, triethylenediamine, tribenzylamine, etc.)

Among the fluorine-containing ether (I), those represented by the formula (I′) are the novel compounds.

Specific examples of the novel fluorine-containing ether are $CH_3CHClOCH_2CH_2F$, $CH_3CHClOCH_2CF_2H$, $CH_3CHClCH_2CF_3$, $CH_3CHClOCH_2CF_2CF_2H$, $CH_3CHClOCH_2C_2F_5$, $CH_3CHClOCH_2C_3F_7$, $CH_3CHClOCH_2CH_2CF_3$, $CH_3CHClOCH_2CH_2C_2F_5$, $CH_3CHClOCH_2CH_2C_3F_7$, $CH_3CHClOCH_2CH(CF_3)_2$, $CH_3CHClOCH(CF_3)_2$, $CH_3CHClOCH_2CF_2CHFCF_3$, $CH_3CHBrOCH_2CH_3$ and ethers analogous to these ethers in which the chlorine atom is replaced with the bromine atom or vice versa (e.g. $CH_3CHBrOCH_2CF_3$, $CH_3CHBrOCH_2C_2F_5$, $CH_3CHBrOCH_2CF_2CHF_2$, etc.).

The fluorine-containing ether (I′) can be dehydrogen halogenated in the same manner as in the case of the fluorine-containing ether (I) to give a corresponding fluorine-containing vinyl ether.

The fluorine-containing vinyl ether (V) is used as a monomer of various fluoropolymers including fluororesins and fluoroelastomers. The fluorine-containing vinyl ether (V) can be copolymerized with an ethylenically unsaturated compound (e.g. ethylene, propylene, vinyl chloride, etc.) or an ethylenically unsaturated fluorine-containing compound (e.g. tetrafluoroethylene, chlorotrifluoroethylene, 1,1-difluoroethylene, etc.) to give a fluororesin which is useful as a polymer component in a cold setting coating

or a fluoroelastomer.

The fluorine-containing vinyl ether (V) can be copolymerized with a fluoroethylene (e.g. tetrafluoroethylene, chlorotrifluoroethylene, etc.) and a hydroxyl or epoxy group-containing vinyl ether (e.g. hydroxybutyl vinyl ether) to give a terpolymer, which is useful as a polymer component in a cold setting coating with improved resistances to weather, heat, dirt, chemicals and the like (cf. Japanese Patent Kokai Publication No. 67518/1985).

Above all, a terpolymer comprising the fluorine-containing vinyl ether (V) having a relatively short fluorine-containing aliphatic group such as a group of the formula: $Z(CF_2)_r$- in which Z is a hydrogen atom or a fluorine atom and r is 1, 2 or 3, for example, a terpolymer of the fluorine-containing vinyl ether (V), chlorotrifluoroethylene (CTFE) and hydroxybutyl vinyl ether (HBVE) has good solubility in the solvent and compatibility with the hydrocarbon base polymers. Particularly, the terpolymer of the fluorine-containing vinyl ether, CTFE and HBVE in which the fluorine-containing vinyl ether is 2,2,2-trifluoroethyl vinyl ether (3FVE), 2,2,3,3-tetrafluorobutyl vinyl ether (4FVE) which is derived from 1-chloroethyl 2,2,3,3-tetrafluoropropyl ether or 2,2,3,3,4,4-hexafluoropropyl vinyl ether (6FVE) has better compatibility with the methyl methacrylate base polymer and more homogeneously compounded therewith than a terpolymer comprising the fluorine-containing vinyl ether having a longer fluoroalkyl group such as a group of the formula: $Z(CF_2)_r$- in which Z is the same as defined above and r is an integer of 4 to 9. Therefore, the cold setting coating composition having better properties can be prepared. The compatibility of various terpolymers with the methyl methacrylate base polymers (terpolymers of methyl methacrylate, ethyl methacrylate and hydroxyethyl methacrylate (MMA/EMA/HEMA)) are shown in Table 1.

Table

| Fluoropolymer*1) | Methacrylate base terpolymer Molar ratio of MMA/EMA/HEMA | | |
|---|---|---|---|
| F-containing vinyl ether*2) | 70/20/10 | 80/10/10 | 90/0/10 |
| 3FVE | O*3) | O | Δ*4) |
| 4FVE | O | Δ | Δ |
| 6FVE | Δ | Δ | O |
| 8FVE | ×*5) | × | × |
| 16FVE | × | × | × |
| Note: | | | |

*1) Molar ratio of the fluorine-containing vinyl ether/CTFE/HBVE = 40/50/10.

*2) 3FVE: $CF_3CH_2OCH=CH_2$

4FVE: $H(CF_2)_2CH_2OCH=CH_2$

6FVE: $H(CF_2)_3CH_2OCH=CH_2$

8FVE: $H(CF_2)_4CH_2OCH=CH_2$

16FVE: $H(CF_2)_8CH_2OCH=CH_2$

*3) O: Homogeneously dissolved.

Δ: Slightly unhomogeneously dissolved but formed a uniform coating.

×: Phase separation into two phases or clouded and formed no uniform coating.

By the compounding of the fluoropolymer, the resistance to chemicals, weather and dirt of the methyl methacrylate base polymer are improved (Japanese Patent Kokai Publication No. 189108/1984).

2,2,2-Trifluoroethyl vinyl ether (fluoxene) which is produced from 1-chloroethyl 2,2,2-trifluoroethyl ether through dehydrogen chloride is used as an anesthetic and also useful as a monomer. For example, a homopolymer of 2,2,2-trifluoroethyl vinyl ether is a thermoplastic polymer having good thermal stability (cf. U.S. Patent No. 2,813, 847). A copolymer of 2,2,2-trifluoroethyl vinyl ether with a vinyl ester type compound is used to form a flexible film or a soft fluoroelastomer (cf. U.S. Patent Nos. 2,851,449 and 3,025,279), and a copolymer with tetrafluoroethylene is usable as a solvent resistant fluoroelastomer (cf. U.S. Patent No. 2,991,278).

Similarly, 2,2,3,3,3-pentafluoropropyl vinyl ether which is derived from 1-chloroethyl 2,2,3,3,3-pentafluoropropyl ether can be used as a fluoroelastomer or a polymer component in the coating composition and impart water or oil resistance to a substrate.

## PREFERRED EMBODIMENTS OF THE INVENTION

The present invention will be illustrated by the following Examples.

### Example 1

Preparation of 1-chloroethyl 2,2,3,3-tetrafluoropropyl ether

In a 500 ml flask equipped with a reflux condenser, a thermometer and a stirrer, 2,2,3,3-tetrafluoropropanol (132 g, 1.0 mole) and paraldehyde (52.8 g, 0.33 mole) were mixed and cooled to a temperature of 5 to 10 °C on an ice bath. At that temperature, absolute hydrogen chloride was bubbled in the mixture at a flow rate of 0.209 g/min. for 3.5 hours (total mount of 43.8 g) under atmospheric pressure while stirring. The reaction product was dried over anhydrous sodium sulfate and fractionally distilled to recover 1-chloroethyl 2,2,3,3-tetrafluoropropyl ether ($CH_3CHClOCH_2CF_2CF_2H$) (158 g) at 66 °C/100 mmHg. Yield: 81 %.

MS (m/e): 194 (p), 179 (p-$CH_3$), 159 (p-Cl), 63 ($CH_3CClH$-), 51 ($CHF_2$-).

$^1$H-NMR: $\delta$ (ppm) = 5.98 (1H, tt, J = 52.4, 4.8 Hz), 5.76 (1H, q, J = 5.4 Hz), 4.33-3.93 (2H, m, $J_{gemHH}$ = 12.0, $J_{HF}$ = 12.0 Hz), 1.83 (3H, d, J = 5.5 Hz).

$^{19}$F-NMR: $\delta$ (ppm) = 46.8 (2F, m), 61.0-61.2 (2F, m, $J_{gemFF}$ = 313.4, $J_{HF}$ = 52.5 Hz).

### Example 2

Preparation of 1-chloroethyl 2,2,2-trifluoroethyl ether

In the same manner as in Example 1 but using 2,2,2-trifluoroethanol (100 g, 1.0 mole) as the fluorine-containing alcohol, the reaction was carried out to recover 1-chloroethyl 2,2,2-trifluoroethyl ether ($CH_3CHClOCH_2CF_3$) (127 g) at 81 °C/760 mmHg. Yield: 78 %.

MS (m/e): 162 (p), 147 (p-$CH_3$), 127 (p-Cl), 69 ($CF_3$).

$^1$H-NMR: $\delta$ (ppm) = 5.71 (1H, q, J = 5.7 Hz), 4.16-3.89 (2H, m $J_{HF}$ = 8.8 Hz), 1.81 (3H, d, J = 5.1 Hz).

$^{19}$F-NMR: $\delta$ (ppm) = -4.3 (3F, t, J = 8.5 Hz).

### Example 3

Preparation of 1-chloroethyl 2,2,3,3,3-pentafluoropropyl ether

In the same manner as in Example 1 but using 2,2,3,3,3-pentafluoropropanol (150 g) as the fluorine-containing alcohol, the reaction was carried out to recover 1-chloroethyl 2,2,3,3,3-pentafluoropropyl ether ($CH_3CHClOCH_2C_2F_5$) (168 g) at 45 °C/25 mmHg. Yield: 79 %.

MS (m/e): 212 (p), 197 (p-$CH_3$), 177 (p-Cl), 69 ($CF_3$).

$^1$H-NMR: $\delta$ (ppm) = 5.67 (lH, q, J = 5.5 Hz), 4.48-3.72 (2H, m $J_{HF}$ = 13.2, $J_{HH}$ = 13.2 Hz), 1.78 (3H, d, J = 5.28 Hz).

$^{19}$H-NMR: $\delta$ (ppm) = 6.8 (3F, m), 46.4 (2F, tm, $J_{HF}$ = 13.2 Hz).

### Example 4

In the same manner as in Example 1 but using the fluorine-containing acetal $CH_3CH(OCH_2CF_2CHF_2)_2$ -

(145 g, 0.5 mole) in place of 2,2,3,3-tetrafluoropropanol, the reaction was carried out to obtain $CH_3CHClOCH_2CF_2CHF_2$ (310 g). Boiling point: 66 °C/100 mmHg. Yield: 80 %.

Examples 5-9

In the same manner as in Example 1 but using the fluorine-containing alcohol described below in place of 2,2,3,3-tetrafluoropropanol, the reaction was carried out to obtain the below described fluorine-containing ether in the described yield.

(Example 5)

Alcohol: $H(CF_2CF_2)_3CH_2OH$ (332 g)
Ether: $CH_3CHClOCH_2(CF_2CF_2)_3H$ (78 %)
(Colorless liquid)

(Example 6)

Alcohol: $CF_3CHFCF_2CH_2OH$ (182 g)
Ether: $CH_3CHClOCH_2CF_2CHFCF_3$ (81 %)
(Liquid)

(Example 7)

Alcohol: $C_8F_{17}CH_2CH_2OH$ (464 g)
Ether: $CH_3CHClOCH_2CH_2C_8F_{17}$ (75 %)

(Example 8)

Alcohol: $(CF_3)_2CHOH$ (168 g)
Ether: $CH_3CHClOCH(CF_3)_2$ (76 %)
(Colorless liquid)

(Example 9)

Alcohol: $F[CF(CF_3)CF_2O]_2CF_2CF_2CH_2OH$ (468 g)
Ether: $CH_3CHClOCH_2CF_2CF_2[OCF_2CF(CF_3)]_2F$ (77 %)

Examples 10-16

In the same manner as in Example 4 but using the fluorine-containing acetal described below in place of $CH_3$ $CH(OCH_2CF_2CHF_2)_2$, the reaction was carried out to obtain the below described fluorine-containing ether in the described yield.

(Example 10)

Acetal: $CH_3CH(OCH_2CF_3)_2$ (226 g)
Ether: $CH_3CHClOCH_2CF_3$ (81 %)
(B.P. 81 °C)

(Example 11)

Acetal: $CH_3CH[OCH_2(CF_2CF_2)_3H]_2$ (690 g)
Ether: $CH_3CHClOCH_2(CF_2CF_2)_3H$ (79 %)
(Colorless liquid)

(Example 12)

Acetal: $CH_3CH(OCH_2C_2F_5)_2$ (326 g)
Ether: $CH_3CHClOCH_2C_2F_5$ (80 %)
(B.P. 45° C/25 mmHg)

(Example 13)

Acetal: $CH_3CH(OCH_2CH_2C_8F_{17})_2$ (954 g)
Ether: $CH_3CHClOCH_2CH_2C_8F_{17}$ (73 %)

(Example 14)

Acetal: $CH_3CH(OCH_2CF_2CHFCF_3)_2$ (390 g)
Ether: $CH_3CHClCH_2CF_2CHFCF_3$ (77 %)
(Colorless liquid)

(Example 15)

Acetal: $CH_3CH[OCH_2CF_2CF_2[OCF_2CF(CF_3)]_2F]_2$ (962 g)
Ether: $CH_3CHClOCH_2CF_2CF_2[OCF_2CF(CF_3)]_2F$ (71 %)

(Example 16)

Acetal: $CH_3CH[OCH(CF_3)_2]_2$ (362 g)
Ether: $CH_3CHClOCH(CF_3)_2$ (76 %)
(Colorless liquid)

Examples 17-21

In the same manner as in Example 1 but using the fluorine-containing alcohol described below and bubbling hydrogen bromide gas at a flow rate of 0.338 g/min. for 4 hours (total amount of 97.2 g), the reaction was carried out to obtain the below described fluorine-containing ether in the described yield.

(Example 17)

Alcohol: $CF_3CH_2OH$ (100 g)
Ether: $CH_3CHBrOCH_2CF_3$ (79 %)
B.P. = 65° C/200 mmHg.

(Example 18)

Alcohol: $C_2F_5CH_2OH$ (150 g)
Ether: $CH_3CHBrOCH_2C_2F_5$ (75 g)
B.P. = 53° C/100 mmHg.

(Example 19)

Alcohol: $CHF_2CF_2CH_2OH$ (132 g)
Ether: $CH_3CHBrOCH_2CF_2CF_2H$ (77 %)
B.P. = 48° C/10 mmHg.

(Example 20)

Alcohol: $H(CF_2CF_2)_3CH_2OH$ (332 g)

Ether: $CH_3CHBrOCH_2(CF_2CF_2)_3H$ (73 %)
(Colorless liquid)

(Example 21)

Alcohol: $C_8F_{17}CH_2CH_2OH$ (464 g)
Ether: $CH_3CHBrOCH_2CH_2C_8F_{17}$ (71 %)

## Claims

1. A process for preparing a fluorine-containing ether of the formula:

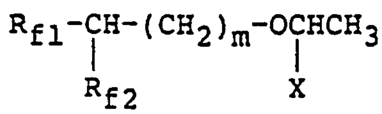

(I)

wherein X is a chlorine atom or a bromine atom, and $R_{f1}$ and $R_{f2}$ are the same or different and a hydrogen atom, a fluorine atom or a fluorine-containing $C_1$-$C_{20}$ aliphatic or ether group, m is an integer of 0 to 9 provided that at least one of $R_{f1}$ and $R_{f2}$ is not a hydrogen atom, and when m is zero (0), neither of $R_{f1}$ and $R_{f2}$ is a fluorine atom, which process comprising reacting at least one compound selected from the group consisting of a fluorine-containing alcohol of the formula:

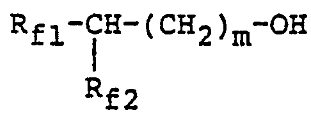

(II)

or a fluorine-containing acetal of the formula:

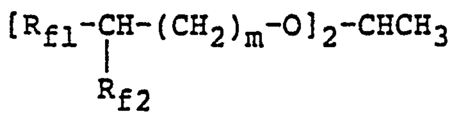

(III)

wherein $R_{f1}$, $R_{f2}$ and m are the same as defined above with acetaldehyde and at least one hydrogen halide selected from the group consisting of hydrogen chloride and hydrogen bromide.

2. The process according to claim 1, wherein the fluorine-containing alcohol is used as one of the starting materials.

3. The process according to claim 2, wherein the fluorine-containing alcohol is at least one selected from the group consisting of $CH_2FCH_2OH$, $CHF_2CH_2OH$, $CF_3CH_2OH$, $CF_3CH_2CH_2OH$, $C_2F_5CH_2OH$, $C_3F_7CH_2OH$, $C_2F_5CH_2CH_2OH$, $C_3F_7CH_2CH_2OH$, $C_8F_{17}CH_2CH_2OH$, $CHF_2CF_2CH_2OH$, $H(CF_2CF_2)_2CH_2OH$, $H(CF_2CF_2)_3CH_2OH$, $H(CF_2CF_2)_4CH_2OH$, $H(CF_2CF_2)_5CH_2OH$, $CF_3CHFCF_2CH_2OH$, $(CF_3)_2CHOH$, $(CF_3)_2CHCH_2OH$, $(CF_3)_2CFCF_2CF_2CH_2CH_2OH$, $(CF_3)_2CFCH_2OH$, $CF_3CF_2CF_2OCF_2CF_2CH_2OH$, $F[CF(CF_3)CF_2O]_2CF_2CF_2CH_2OH$, $F[CF(CF_3)CF_2O]_3CF_2CF_2CH_2OH$, $C_3F_7OCF_2CF_2CF_2OCF_2CF_2CH_2OH$ and $F(CF_2CF_2CF_2O)_3CF_2CF_2CH_2OH$.

4. The process according to claim 1, wherein the fluorine-containing acetal is used as one of the starting materials.

5. The process according to claim 4, wherein the fluorine-containing acetal is at least one selected from the group consisting of $CH_3CH(OCH_2CH_2F)_2$, $CH_3CH(OCH_2CF_3)_2$, $CH_3CH(OCH_2C_2F_5)_2$, $CH_3CH(OCH_2CF_2CHF_2)_2$, $CH_3CH(OCH_2CF_2CF_2CF_2CF_2H)_2$, $CH_3CH[OCH_2(CF_2CF_2)_3H]_2$, $CH_3CH[OCH_2(CF_2CF_2)_5H]_2$, $CH_3CH(OCH_2CF_2CHFCF_3)_2$, $CH_3CH[OCH(CF_3)_2]_2$, $CH_3CH(OCH_2C_3F_7)_2$, $CH_3CH(OCH_2CH_2C_2F_5)_2$, $CH_3CH(OCH_2CH_2C_8F_{17})_2$, $CH_3CH[OCH_2CH_2CF_2CF_2CF(CF_3)_2]_2$, $CH_3CH[OCH(CF_3)_2]_2$,

$CH_3CH(OCH_2CF_2CF_2OC_3F_7)_2$, $CH_3CH$-$[OCH_2CF_2CF_2OCF_2CF(CF_3)OCF_2CF_2(CF_3)]_2$, $CH_3CH$-$[OCH_2CF_2CF_2OCF_2CF(CF_3)OCF_2CF(CF_3)OCF_2CF_2(CF_3)]_2$, $CH_3CH$-$(OCH_2CF_2CF_2OCF_2CF_2CF_2OC_3F_7)_2$ and $CH_3CH$-$(OCH_2CF_2CF_2OCF_2CF_2CF_2OCF_2CF_2CF_2OC_3F_7)_2$.

6. A fluorine-containing ether of the formula:

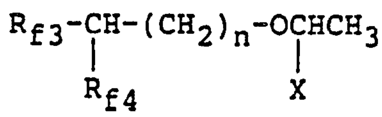

(I')

wherein X is a chlorine atom or a bromine atom, and $R_{f3}$ and $R_{f4}$ are the same or different and a hydrogen atom, a fluorine atom or a fluorine-containing $C_1$-$C_3$ aliphatic group, n is an integer of 0 to 3 provided that at least one of $R_{f3}$ and $R_{f4}$ is not a hydrogen atom and the total number of the carbon atoms in $R_{f3}$ and $R_{f4}$ is not larger than 3.

7. The fluorine-containing ether according to claim 7, wherein X in the formula (I′) is a chlorine atom.

8. The fluorine-containing ether according to claim 7, wherein X is the formula (I′) is a bromine atom.

European Patent Office

# EUROPEAN SEARCH REPORT

Application Number

EP 88 12 1136

## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| A | HOUBEN-WEYL: "Methoden der organischen Chemie" vol. VI, 4th edition, 1965, pages 125-129, part 3; GEORG THIEME VERLAG, Stuttgart * page 128, lines 1-2 * | 1 | C 07 C 43/12<br>C 07 C 41/00 |
| A | US-A-2 888 490 (WALTER) * claim 1 * | 1 | |
| D,A | US-A-3 658 918 (JAEGER) * claim 1 * | 1 | |
| D,A | GB-A- 739 731 (MINNESOTA MINING & MANUFACTURING CO.) * claim 1 * | 1 | |
| A | GB-A-1 142 306 (PITTMAN) * page 1, lines 40-42 * | 1 | |
| | | | **TECHNICAL FIELDS SEARCHED (Int. Cl.3)** |
| | | | C 07 C 43/00<br>C 07 C 41/00 |

The present search report has been drawn up for all claims

| Place of search | Date of completion of the search | Examiner |
|---|---|---|
| BERLIN | 22-02-1989 | PROBERT C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)